# EUROPÄISCHE PATENTANMELDUNG

(11) **EP 0 856 734 A2**
(43) Veröffentlichungstag der Anmeldung: **05.08.1998**
(21) Anmeldenummer: 97121699.9
(22) Anmeldetag: 10.12.1997
(51) Int. Cl.: G01N 33/00, G01N 27/22, G01N 27/40

(54) **Sensor zum selektiven Nachweis von Ammoniak in NOx-haltigen sauerstoffreichen Gasen**

(30) Priorität: 01.02.1997 DE 19703796
(71) Anmelder: DORNIER GmbH, 88039 Friedrichshafen (DE)
(72) Erfinder: Plog, Carsten, Dr., 88677 Markdorf (DE); Maunz, Werner, 88677 Markdorf (DE); Müller, Ralf, 88326 Aulendorf (DE)
(74) Vertreter: Meel, Thomas

(57) **Zusammenfassung**

Die Erfindung betrifft einen Sensor zum selektiven Nachweis von Ammoniak in sauerstoffreichem, NOₓ-haltigem Gas. Er umfaßt
- ein als Kondensator wirkendes Bauelement (**20**) und
- eine gasdurchlässige, sensitive Schicht (**30**) als Dielektrikum, wobei die sensitive Schicht (**30**) ein hydrophober, edelmetallfreier Zeolith mit geringer Azidität ist, der eine geordnete, kristalline Struktur aus Primärporen aufweist, deren Durchmesser in der Größenordnung des gaskinetischen Durchmessers von NH₃ ist.

## Beschreibung

Die Erfindung betrifft einen Sensor zum selektiven Nachweis von Ammoniak (NH₃) in NOₓ-haltigen, sauerstoffreichen Gasen, z.B. Abgase von Dieselmotoren oder eines direkteinspritzenden Ottomotors oder die Abluft eines Kraftwerkes ist.

Es ist bekannt, daß die Entstickung von dieselmotorischen Abgasen durch die selektive katalytische Reduktion (SCR) unter Verwendung von Ammoniak als Reduktionsmittel durchgeführt werden kann. Ammoniak kann entweder direkt oder in Form einer Verbindung, aus der Ammoniak on Board gewonnen wird, im Fahrzeug mitgeführt werden.

Für die katalytische Reduktionsreaktion wird Ammoniak dem motorischen Abgas in einem festen Verhältnis zum momentan im Abgas vorhanden NOx-Gehalt zudosiert. Es ist bekannt, daß das NH₃-NOₓ-Verhältnis zum Erreichen des maximal möglichen NOₓ-Umsatzes gerade 1 sein müßte. Ein kleineres Verhältnis führt zu einem geringeren Umsatz, ein größeres Verhältnis führt zu einem "NO₃-Durchbruch". Da weder fahrzeugtaugliche NOₓ- noch NH₃-Sensoren zur Verfügung stehen, werden die NOₓ-Werte aus Motorkennfeldern entnommen, die in Speichern abgelegt wurden.

Auf der einen Seite muß eine NH₃-Emission in allen Betriebszuständen sicher verhindert werden. Auf der anderen Seite gelten NOₓ-Kennfelder nicht für den individuellen Motor, sondern für die Baureihe, so daß herstellungsbedingte Schwankungen im NOₓ-Rohgasgehalt bei gleichen Kennfeldpunkten vorkommen. Außerdem kann der momentane Katalysatorzustand (Temperatur, NOₓ- und NHe-Beladung) bei gleichen Kennfeldpunkten verschieden sein. Deshalb wird in der Praxis ein NH₃/NOₓ-Verhältnis deutlich kleiner als 1 (z.B. 0,6) gewählt, damit ein genügend großer Sicherheitsabstand eingehalten werden kann. Man nimmt also bewußt eine schlechtere Entstickungsrate des Katalysators in Kauf.

Um die NOₓ-Grenzwerte zu erreichen, muß man die NO-Rohemission durch innermotorische Maßnahmen weiter herabsetzen, als es bei besserer Ausnutzung des Katalysators nötig wäre. Dies geht zu Lasten des Dieselverbrauchs.

Eine bessere Ausnutzung des Entstickungskatalysators könnte mit Hilfe eines NH₃-Sensors erreicht werden, der als Regelglied oder als NH₃-Durchbruchssensor im Bereich des Katalysatorendes angebracht würde. Dieser NH₃-Sensor müßte unter dieselmotorischen Abgasbedingungen geringe Menge NH₃ sicher und ohne wesentliche Querempfindlichkeit zu den anderen relevanten Gasen (vor allem NOₓ, H₂O und O₂) nachweisen.

In **EP 0426 989 B1** wird ein chemischer Sensor für Gase auf der Basis zeolithbeschichteter, direkt geheizter, planarer Interdigitalkondensatoren (im folgenden mit IDK abgekürzt) beschrieben. Dieser Sensor besteht in einer Ausführungsform aus einer platinhaltigen, zeolithischen Schicht.

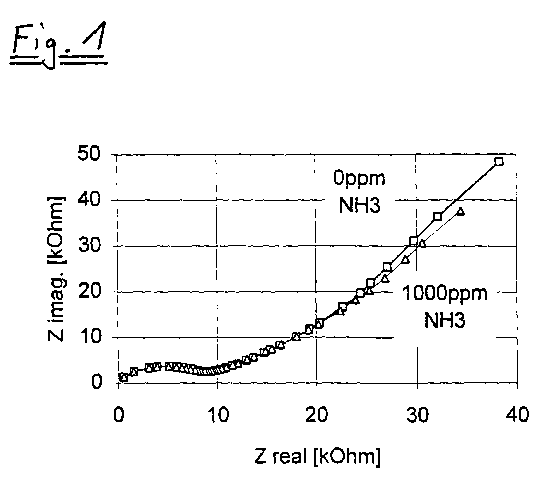

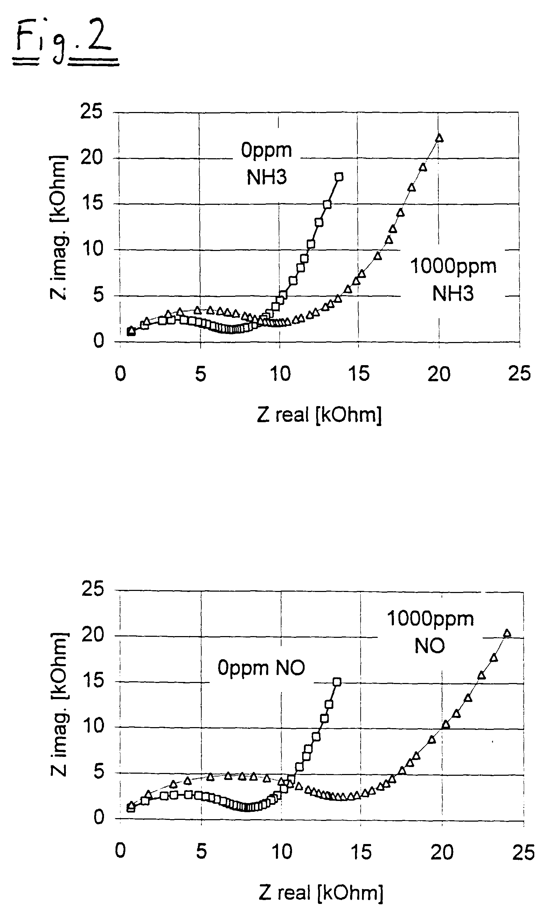

Es zeigt sich nun bei den Untersuchungen zur NH₃-Empfindlichkeit, daß IDK-Sensoren, die mit edelmetallhaltigen Zeolithen beschichtet sind, aus zwei Gründen für einen Einsatz als NH₃-Sensor im Abgas nicht in Frage kommen: entweder fehlt ihnen überhaupt eine ausreichend große NH₃-Empfindlichkeit (Fig. 1) oder sie weise zwar NH₃ nach (Fig. 2 oben), besitzen aber gleichzeitig eine sehr hohe Querempfindlichkeit zu NO (Fig. 2 unten).

Die Fig. 1 zeigt die Impedanzspektren (Frequenzbereich: 20 Hz bis 1 MHz) eines IDK-Sensors mit einer PtNaZSM5-Schicht mit (dreieckige Symbole) und ohne (rechteckige Symbole) NH₃. Im gesamten Frequenzbereich ist der Unterschied zwischen dem Impedanzspektrum mit 1000 ppm NH₃ und 0 ppm NH₃ sehr gering: dieser Sensor zeigt keine NH₃-Empfindlichkeit.

Fig. 2 zeigt oben die Impedanzspektren des bekannten IDK-Sensors mit einer PtNaY-Schicht mit (dreieckige Symbole) und ohne (rechteckige Symbole) NH₃. Das Impedanzspektrum mit 1000 ppm ist im Vergleich zu dem bei 0 ppm NH₃ deutlich zu niedrigeren Frequenzen hin verschoben: Dieser Sensortyp ist empfindlich auf NH₃.

Fig. 2 zeigt unten die Impedanzspektren dieses IDK-Sensors mit und ohne NO. Hier zeigt sich, daß dieser Sensortyp ebenfalls empfindlich auf NO ist. Diese Querempfindlichkeit zu NO läßt einen Einsatz als NH₃-Sensor im Abgas nicht zu.

Aufgabe der Erfindung ist es, einen Sensor zu schaffen, der auch geringe Mengen von NH₃ sicher und ohne wesentliche Querempfindlichkeit zu den anderen relevanten Gasen (vor allem NOₓ, H₂O und O₂) ermöglicht.

Diese Aufgabe wird mit dem Sensor nach Anspruch 1 gelöst. Vorteilhafte Ausführungen der Erfindung sind Gegenstand von Unteansprüchen.

Erfindungsgemäß umfaßt der NH₃-Sensor ein als Kondensator wirkendes Bauelement und eine gasdurchlässige, sensitive Schicht als Dielektrikum, wobei die sensitive Schicht ein hydrophober, edelmetallfreier Zeolith mit geringer Azidität (Säurestärke) ist, der eine geordnete, kristalline Struktur aus Primärporen aufweist, deren Durchmesser in der Größenordnung des gaskinetischen Durchmessers von NH₃ ist.

Die Wechselwirkung des zu detektierenden Moleküls mit dem edelmetallfreien zeolithischen Festkörper des erfindungsgemäßen Sensors geschieht völlig anders als bei dem in der EP 0 426 989 B1 beschriebenen Sensor mit platinhaltiger zeolithischer Schicht. Bei der platinhaltigen zeolithischen Schicht kommt es zu einer katalytischen Umsetzung der Gasmoleküle an den Platinclustern innerhalb des zeolithischen Porensystems. Durch die zeitlich nicht unendlich schnell ablaufenden Reaktion kommt es zu einer Behinderung der Beweglichkeit der Kationen (im allgemeinen Natriumionen) des Zeolithen, die bei erhöhter Temperatur durch das elektrische Wechselfeld bewegt werden können. Diese Erniedrigung der Mobilität der Kationen macht sich bei niedrigen Frequenzen in einer Veränderung der Impedanz bemerkbar.

Bei dem erfindungsgemäßen Sensor mit edelmetallfreier, hydrophober Zeolithschicht als ammoniaksensitive Schicht finden keine katalytische Umsetzung des NH₃ statt, da die in Frage kommenden Zeolithe keinerlei katalytische Umsetzungsaktivität für NH₃ besitzen. Vielmehr spielt die Adsorption des Ammoniakmoleküls die bestimmende Rolle für das Sensorverhalten. Die Protonen der adsorbierten Ammoniakmoleküle sind die Ladungsträger, deren Mobilität im elektrischen Wechselfeld das Sensorsignal erzeugen. Gegenüber dem unbelegten Ausgangszustand ändert sich die ionische Leitfähigkeit im gesamten untersuchten Frequenzbereich von 20 Hz bis 1 MHz. Bei den hohen Frequenzen können nur noch die Protonen dem Wechselfeld folgen, die wesentlich schwereren anderen, in Frage kommenden Kationen können das nicht mehr.

Der hydrophobe Charakter der Zeolithe wird durch ihre Tendenz bestimmt, polare Moleküle (z.B. Wasser) nur in sehr geringen Mengen, unpolare Verbindungen (z.B. Kohlenwasserstoffe) hingegen in großen Mengen aufzunehmen. Da bei der Signalerzeugung des erfindungsgemäßen NH₃-Sensors der Adsorptionszustand des NH₃-Moleküls im zeolithischen Porensystem die entscheidende Rolle spielt, ist es wichtig, daß nur geringe eine Menge an Wasser im Porensystem vorliegt. Auf der einen Seite könnte sich das NH₃ im Porenwasser als Ammoniumhydroxid (NH₄OH) lösen und zum Teil dissoziieren. Auf der anderen Seite wäre die Querempfindlichkeit zum Wasser groß.

Die Erfindung wird anhand von Fig. näher erläutert. Es zeigen:
- Fig. 1: Impedanzspektren (Frequenzbereich: 20 Hz bis 1 MHz) eines mit PtZSM5 beschichteten, an sich bekannten IDK-Sensors, beaufschlagt mit 1000 ppm NH₃ (dreieckige Symbole) und 0 ppm NH₃ (rechteckige Symbole) in 10 % O₂, 5 % abs H₂O.
- Fig. 2: Impedanzspektren eines mit PtY-Zeolith beschichteten, an sich bekannten IDK-Sensors in 10% O₂, 5% abs H₂O;
oben: bei 1000 ppm NH₃ (dreieckige Symbole) und 0 ppm NH₃ (rechteckige Symbole)
unten: bei 1000 ppm NO (dreieckige Symbole) und 0 ppm NO (rechteckige Symbole);
- Fig. 3: oben: relative Widerstandsänderungen dR/R1 (weiße Balken) und relative Kapazitätsänderungen dC/C1 (dunkle Balken) für unterschiedliche Modifikationen des Zeolithtyps Beta bei einer NH₃-Konzentrationsänderung von 0 auf 124 ppm (10 % O₂, 5 % abs H₂O, 251 ppm NO)
unten: Widerstandsänderung dR2 (weiße Balken) und Kapazitätsänderung dC2 (dunkle Balken) bei einer NO-Konzentrationsänderung von 251 auf 653 ppm (10 % O₂, 5 % abs H₂O, 124 ppm NH₃) im Verhältnis zu der Widerstandsänderung dR1 bzw. dC1 bei einer NH₃-Konzentrationsänderung von 0 auf 124 ppm (10 % O₂, 5 % abs H₂O, 251 ppm NO)

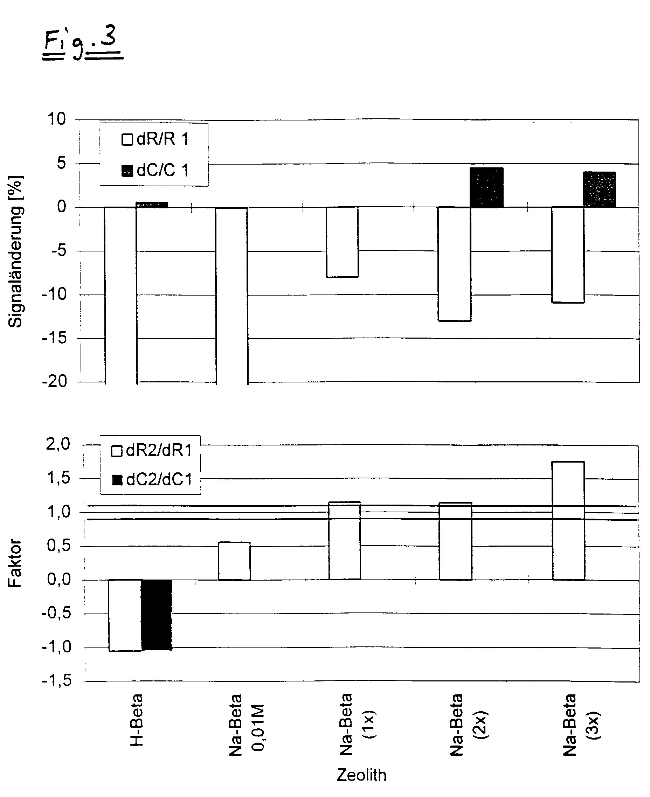

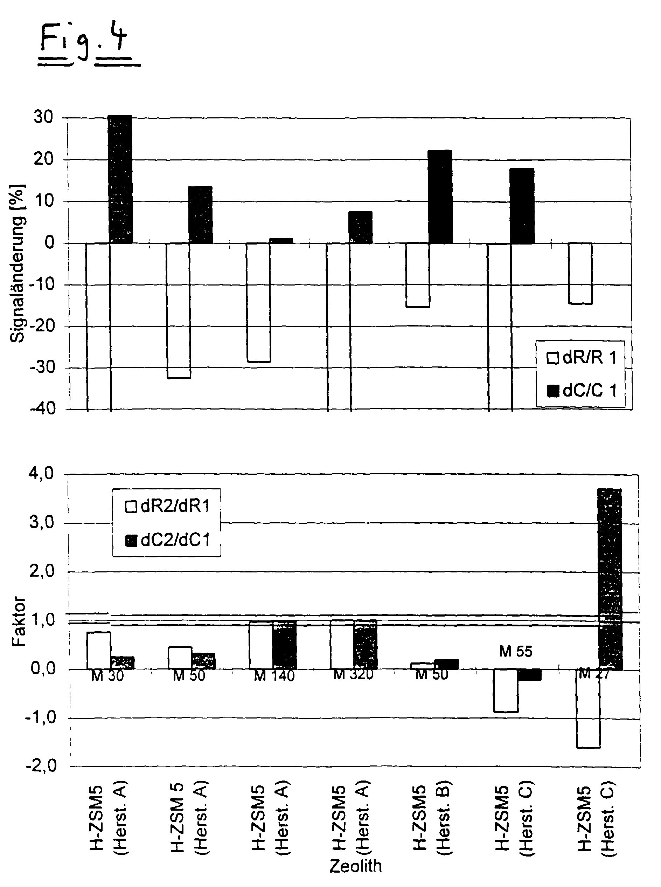

- Fig. 4: oben: relative Widerstandsänderungen dR/R1 (weiße Balken) und relative Kapazitätsänderungen dC/C1 (dunkle Balken) für unterschiedliche Moduli des Zeolithtyps ZSM5 bei einer NH₃-Konzentrationsänderung von 0 auf 124 ppm (10 % O₂, 5 % abs H₂O, 251 ppm NO)
unten: Widerstandsänderung dR2 (weiße Balken) und Kapazitätsänderung dC2 (dunkle Balken) bei einer NO-Konzentrationsänderungen von 251 auf 653 ppm (10 % O₂, 5 % abs H₂O, 124 ppm NH₃) im Verhältnis zu der Widerstandsänderung dR1 bzw. dC1 bei einer NH₃-Konzentrationsänderung von 0 auf 124 ppm (10 % O₂, 5 % abs H₂O, 251 ppm NO)

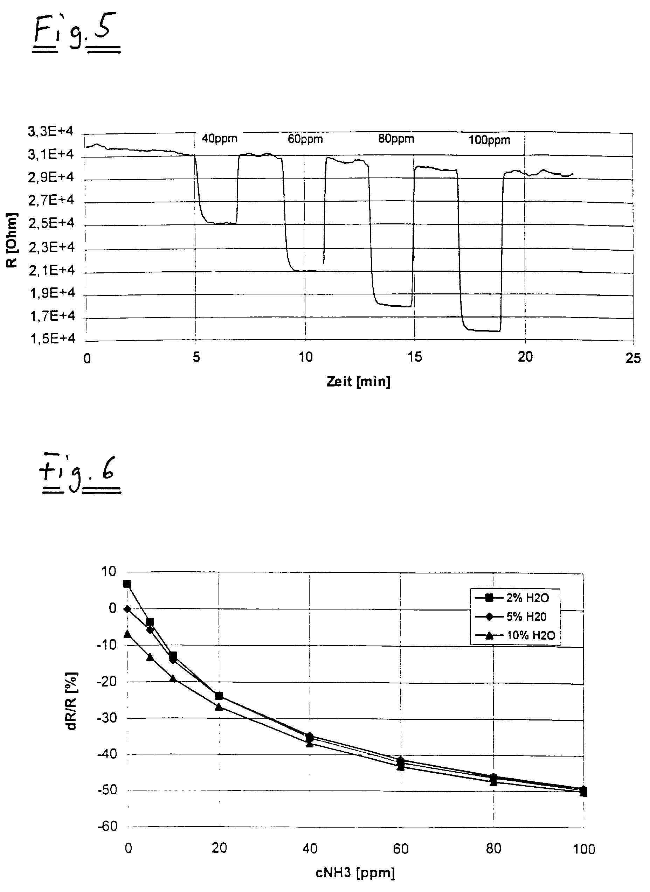

- Fig. 5: Widerstandsänderungen über der Zeit für den Zeolithtyp H-ZSM5 (Modul 140) durch unterschiedliche NH₃-Konzentrationsänderungen (40 ppm, 60 ppm, 80 ppm 100 ppm) in einem Meßgas mit 1000 ppm NO, 5% abs H₂O und 10% O_{2;} Sensorbetriebstemperatur 420 ^{o}C, Meßgastemperatur 260^{o}C

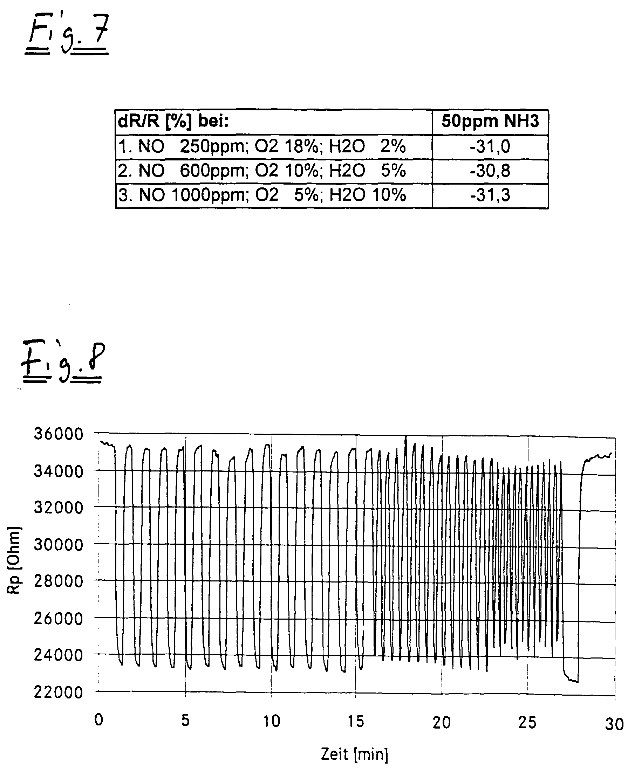

- Fig. 6: Relative Widerstandsänderungen dR/R für den Zeolithtyp H-ZSM5 (Modul 140) durch NH₃-Konzentrationsänderungen 0 - 100 ppm in einem Meßgas mit 10% O₂, 2%H₂O (Quadrat), 5% H₂O (Raute), 10% H₂O (Dreieck) ; Sensorbetriebstemperatur 420 ^{o}C, Meßgastemperatur 260^{o}C

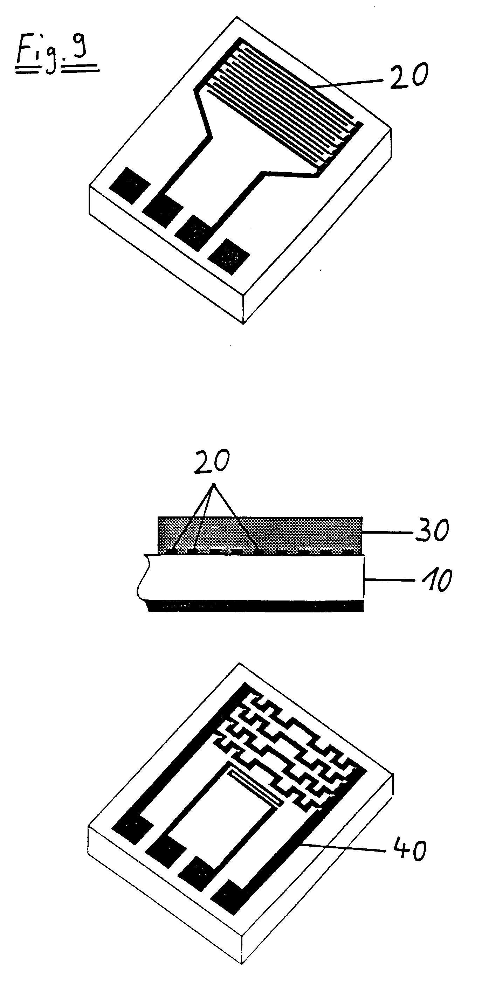

- Fig. 7: Relative Widerstandsänderungen dR/R für den Zeolithtyp H-ZSM5 (Modul 140) durch NH₃-Konzentrationsänderungen von 50 ppm bei unterschiedlichen Betriebspunkten: 1. Leerlauf, 2. mittlere Last, 3. Vollast; Sensorbetriebstemperatur 420 ^{o}C, Meßgastemperatur 260^{o}C
- Fig. 8: Widerstandsänderungen für den Zeolithtyp H-ZSM5 (Modul 140) durch NH₃-Konzentrationsänderungen 0 ppm --->60 ppm ---->0 ppm in einem Meßgas mit 10% O₂, 5% H₂O; Sensorbetriebstemperatur 420 ^{o}C, Meßgastemperatur 260^{o}C;
0 - 16 min: 30 sec 60 ppm NH₃, 30 sec 0 ppm NH₃
16 - 23 min: 20 sec 60 ppm NH₃, 20 sec 0 ppm NH₃
23 - 27 min: 15 sec 60 ppm NH₃, 15 sec 0 ppm NH₃
- Fig. 9: einen erfindungsgemäßen Sensor.

Es wurden umfangreiche Untersuchungen der NH₃-Empfindlichkeit zeolithischer IDK-Sensoren in Abhängigkeit von dem eingesetzten Zeolithtyp und der verwendeten Zeolithmodifikation durchgeführt. Dabei wurde sowohl die NH₃-Sensorempfindlichkeit als auch die Querempfindlichkeit zu NO, H2O, CO, HC untersucht. Die Zeolithe wurden sowohl in der Na- und H-Form eingesetzt. Zusätzlich wurden diese Kationen durch andere Elemente durch Ioneneintausch kationischer Verbindungen dieser Elemente durchgeführt.

Die Fig. 3 zeigt als ein Beispiel die NH₃-Sensorempfindlichkeit (oben) und die NO-Querempfindlichkeit (unten) für Modifikationen des Beta-Zeolithen. Der Modul ist bei allen dargestellten Modifikationen im wesentlichen der gleiche. Die Fig. 4 zeigt als ein weiteres Beispiel die NH₃-Sensorempfindlichkeit (oben) und NO-Querempfindlichkeit (unten) für unterschiedliche Moduli des Zeolithtypen ZSM5 in der H-Form. Dabei wurden Zeolithen unterschiedlicher Hersteller berücksichtigt. Die Hersteller sind in der Fig. 4 mit "Herst. A", "Herst. B", "Herst. C" bezeichnet.

Bei den Diagrammen zur NO-Querempfindlichkeit ist zu beachten, daß bei einem Faktor 1,0 gerade kein NO-Einfluß vorliegt. Die Meßsäulen geeigneter Sensormaterialien enden deshalb in dem eingezeichneten Intervall um den Faktor 1,0.

Die Messungen wurden bei 10% O₂ und 5% abs H₂O in der Weise durchgeführt, daß
(1) NH₃ von 0 auf 124 ppm erhöht wurde, wobei sich zusätzlich 251 ppm NO im Meßgas befand,
(2) NO von 251 ppm auf 653 ppm erhöht wurde, wobei sich zusätzlich 124 ppm NH₃ im Meßgas befand.

Durch diese Meßprozedur wurden die tatsächlichen bei einer bestimmten NOₓ-Rohemission nach einer bestimmten Katalysatorlänge (30% der Gesamtlänge) bei einem bestimmten NO-Umsatz (60%) vorliegenden NO/NH₃-Konzentrationen berücksichtigt.

Die Fig. 3 zeigt das bislang noch nicht bekannte Ergebnis, daß sowohl die NH₃-Empfindlichkeit als auch die NO-Querempfindlichkeit beim gleichen Zeolithtyp (im Beispiel "Beta") durch die Art und Konzentration der Austauschkationen und damit durch die Azidität des modifizierten Zeolithen beeinflußt werden kann.

Die Fig. 4 zeigt das bislang noch nicht bekannte Ergebnis, daß sowohl die NH₃-Empfindlichkeit als auch die NO-Querempfindlichkeit bei der gleichen Zeolithform (im Beispiel "H-Form") von der Wahl des Moduls und damit der Azidität des Zeolithen abhängt.

Damit ergibt sich überraschenderweise die Möglichkeit, die Querempfindlichkeit gegenüber NO durch Eintausch unterschiedlich hoher Anteile von Kationen, durch die Art des Kations, durch die Wahl des Zeolithtyps und durch den Modul des Zeolithen, und damit durch die Einstellung der Azidität der Zeolithe, einzustellen.

So ist der H-Beta-Zustand mit einer Widerstandsabnahme von 72% sehr empfindlich für NH₃, gleichzeitig aber auch sehr querempfindlich gegen NO. Durch eine NO-Konzentrationsänderung von 251 auf 653 ppm ändert sich nämlich der Widerstand um einen Faktor -1,1. Dies bedeutet, daß der Widerstand wieder zunimmt und seinen Ausgangswert von der NH₃-Beaufschlagung erreicht. In Fig. 3 stehen die Proben Na-Beta (0,01M), Na-Beta(1x), Na-Beta(2x) und Na-Beta(3x) für eine zunehmende Natriumionenkonzentration, wodurch die Azidität verringert wird. Die Zeolithe Na-Beta(1x) und Na-Beta(2x) zeigen fast keine NO-Querempfindlichkeit. (Faktor 1,0 bedeutet, daß kein NO-Einfluß vorliegt).

Fig. 4 zeigt mehrere Zeolithen des Typs ZSM5, wobei das Modui der einzelnen Zeolithen des gleichen Herstellers von links nach rechts kleiner wird, wodurch die Azidität verringert wird. Aus dieser Fig. ist zu entnehmen, daß z.B ein H-ZSM5 mit einem Modul von 140 empfindlich für NH₃ und nicht querempfindlich gegen NO ist, während ein HZSM5 des gleichen Herstellers mit einem Modul 50 zwar sehr NH₃-empfindlich ist aber gleichzeitig auch eine stake NO-Querempfindlichkeit besitzt.

Fig. 5 zeigt die NH₃-Empfindlichkeit für den Zeolithtyp H-ZSMF (Modul 140) als eine Ausführungsform des erfindungsgemäßen NH₃-Sensors. Der Sensor wird hintereinander mit 40 ppm, 60 ppm, 80 ppm, 100 ppm NH₃ beaufschlagt. Der Widerstand nimmt durch 40 ppm NH₃ von 31 auf 25 kOhm ab. Dies entspricht eine Widerstandsabnahme um 20%. Bei 100 ppm NH₃ nimmt der Widerstand um die Hälfte auf 15 kOhm ab.

Die Fig. 6 zeigt für verschiedene H₂O-Konzentrationen (2%, 5%, 10%) im Feedgas die Abhängigkeit zwischen der Widerstandsabnahme und der NH₃-Konzentration eines erfindungsgemäßen H-ZSM5-Zeoliths mit Modul 140. Nur bei sehr geringen NH₃-Konzentrationen macht sich ein Wechsel der H₂O-Konzentrationen im relativen Wertebereich störend bemerkbar. Ab 40 ppm NH₃ spielt diese Restquerempfindlichkeit gegenüber Wasser keine Rolle mehr.

Die Fig. 7 zeigt in tabellarischer Form die Signaländerung eines erfindungsgemäßen NH₃-Sensors (H-ZSM5, Modul 140), die bei unterschiedlichen Feedgaszusammensetzungen durch 50 ppm NH₃ hervorgerufen werden. Die Feedgaszusammensetzungen entsprechen den Bedingungen bei Leerlauf, Zwischenlast und Vollast nach einer bestimmten Länge eines SCR-Katalysators mit einem bestimmten NOₓ-Umsatz. Es zeigt sich, daß 50 ppm NH₃ in allen Lastzuständen, unabhängig vom jeweiligen Gehalt an NO, O₂, H₂O, sicher erkannt werden können.

Fig. 8 zeigt das Ansprechverhalten eines erfindungsgemäßen NH₃-Sensors anhand der Widerstandsänderung aufgrund periodischer NH₃-Konzentrationsänderungen zwischen 0 und 60 ppm. Die Gasbeaufschlagung wurde in der folgenden Weise durchgeführt:
0 - 16 min: 30 sec 60 ppm NH₃, 30 sec 0 ppm NH₃
16 - 23 min: 20 sec 60 ppm NH₃, 20 sec 0 ppm NH₃
23 - 27 min: 15 sec 60 ppm NH₃, 15 sec 0 ppm NH₃
Der NH₃-Sensor besitzt in dieser Ausführungsform eine T50-Ansprechzeit im Bereich von 2 sec.

Fig. 9 einen erfindungsgemäßen Sensor mit einem Interdigitalkondensator **20** als das als Kondensator wirkende Bauelement sowie der gasdurchlässigen, sensitiven Zeolithschicht **30**. Die obere Abbildung zeigt eine Draufsicht auf den Sensor. In der mittleren Abbildung ist ein Schnitt durch den Sensor dargestellt. Die untere Abbildung zeigt eine weitere Draufsicht auf den Sensor, bei der die Heizerstruktur **40** zu erkennen ist.

Auf einem Substrat **10**, z. B. aus Quarzglas, Si oder Al₂O₃ sind die parallelen Leiterbahnen (z.B aus Au) des Interdigitalkondensators **20** aufgebracht. Sie sind derart verschaltet, daß sich eine Anordnung elektrisch parallel geschalteter Kondensatoren ergibt, wobei ein einzelner Kondensator jeweils zwei benachbarte Leiterbahnen umfaßt. Über und zwischen den Leiterbahnen ist die gasdurchlässige, sensitive Zeolithschicht **30** angeordnet. Die Schichtdicke der Zeolithschicht **30** liegt z.B. in der Größenordnung von 40 µm. Auf der Unterseite des Substrats **10** ist eine Heizung **40** angeordnet, deren Struktur aus einzelnen Heizungsdrähten in der unteren Abbildung im Detail dargestellt ist.

## Patentansprüche

1. Sensor zum selektiven Nachweis von Ammoniak in sauerstoffreichem, NOₓ-haltigem Gas, welcher
- ein als Kondensator wirkendes Bauelement (**20**) und
- eine gasdurchlässige, sensitive Schicht (**30**) als Dielektrikum umfaßt, wobei die sensitive Schicht (**30**) ein hydrophober, edelmetallfreier Zeolith mit geringer Azidität ist, der eine geordnete, kristalline Struktur aus Primärporen aufweist, deren Durchmesser in der Größenordnung des gaskinetischen Durchmessers von NH₃ ist.

2. Sensor nach Anspruch 1, **dadurch gekennzeichnet**, daß die Azidität der sensitiven Schicht (**30**) durch Eintausch unterschiedlich hoher Anteile von Kationen, durch die Art des Kations, durch die Wahl des Zeolithtypes oder durch den Modul des Zeolithen eingestellt wird.

3. Sensor nach Anspruch 1 oder 2, **dadurch gekennzeichnet**, daß die Sauerstoffkonzentration zwischen 20% und 5000 ppm liegt.

4. Sensor nach einem der vorangehenden Ansprüche, **dadurch gekennzeichnet**, daß der Zeolith ein Y-, USY, Mordenit, ZSM5, ZSM 12, Beta, L, SAPO-11, SAPO-34 oder MCM41 ist.

5. Sensor nach Anspruch 4, **dadurch gekennzeichnet**, daß der Zeolith in der reinen H-Form oder der reinen Na-Form vorliegt, oder teilweise oder vollständig mit Alkali-, Erdalkali- oder Übergangselementen ausgetauscht ist.

6. Sensor nach Anspruch 4 oder 5, **dadurch gekennzeichnet**, daß im Gitter des Zeolithen Aluminium teilweise durch Übergangselemente ersetzt ist.

7. Sensor nach einem der vorangehenden Ansprüche, **dadurch gekennzeichnet**, daß das sauerstoffreiche, Noₓ-haltige Gas das Abgas eines Dieselmotors oder eines direkteinspritzenden Ottomotors ist.

8. Sensor nach einem der vorangehenden Ansprüche, **dadurch gekennzeichnet**, daß das sauerstoffreiche, NOₓ-haltige Gas die Abluft eines Kraftwerkes ist.

9. Sensor nach einem der vorangehenden Ansprüche, **dadurch gekennzeichnet**, daß das als Kondensator wirkende Bauteil ein Interdigitalkondensator ist.
